# EUROPÄISCHE PATENTSCHRIFT

(11) **EP 1 808 430 B1**
(45) Veröffentlichungstag und Bekanntmachung des Hinweises auf die Patenterteilung: **06.10.2010**
(21) Anmeldenummer: 07000740.6
(22) Anmeldetag: 16.01.2007
(51) Int. Cl.: C07C 263/10, C07C 265/14

(54) **Verfahren zur Herstellung heller Isocyanate**
Method for producing light-coloured isocyanates
Procédé de fabrication d'isocyanate clair

(30) Priorität: 17.01.2006 DE 102006002157
(43) Veröffentlichungstag der Anmeldung: 18.07.2007
(73) Patentinhaber: Bayer MaterialScience AG, 51368 Leverkusen (DE)
(72) Erfinder: Wershofen, Stefan, Dr., 41065 Mönchengladbach (DE); Hagen, Torsten, Dr., 45257 Esssen (DE); Steffens, Christian, Dr., 50737 Köln (DE); Bolton, Jeffrey, Dr., 40489 Düsseldorf (DE)

(56) Entgegenhaltungen:
- EP-B1- 1 187 808
- DE-A1- 10 301 434
- DATABASE CAPLUS [Online] CHEMICAL ABSTRACTS SERVICE, COLUMBUS, OHIO, US; MANABE, AKIYOSHI ET AL: "Polycarbonates with excellent color tone and durability and their manufacture" XP002435080 gefunden im STN Database accession no. 2000:580011 & JP 2000 230046 A (TEIJIN CHEMICALS LTD., JAPAN) 22. August 2000 (2000-08-22)

## Beschreibung

Die Erfindung betrifft ein Verfahren zur Herstellung von hellen Isocyanaten durch Umsetzung eines Amins oder eines Gemischs aus zwei oder mehr Aminen mit Phosgen, dadurch gekennzeichnet, dass das eingesetzte Phosgen weniger als 100 ppm an Schwefel in elementarer oder gebundener Form enthält.

Isocyanate und Isocyanatgemische werden gemäß dem Stand der Technik durch Phosgenierung der entsprechenden Amine hergestellt. Für Polyurethanschäume finden beispielsweise di- oder polyfunktionelle aromatische Isocyanate der Diphenylmethan-Reihe (MDI) Anwendung. Bedingt durch den Herstellprozess werden nach der Phosgenierung und der anschließenden Aufarbeitung (Abtrennung des Lösemittels; Abtrennen von monomerem MDI) oft dunkel gefärbte Produkte erhalten, die wiederum gelblich verfärbte Polyurethanschäume oder andere, ebenfalls verfärbte Polyurethan(PUR)-Materialien ergeben. Dies ist unerwünscht, da eine solche Färbung den visuellen Gesamteindruck beeinträchtigt und geringfügige Inhomogenitäten hervortreten lässt, z. B. als Schlieren in den erhaltenen Schäumen. Helle Isocyanate, bzw. Isocyanate, die eine reduzierte Menge an farbgebenden Komponenten enthalten, werden deshalb als Rohstoffe bevorzugt.

Es hat daher nicht an Versuchen gefehlt, Isocyanate und insbesondere die Di- und Polyisocyanate der Diphenylmethan-Reihe (MDI) mit heller Farbe zu erhalten. Zur empirischen Farbaufhellung von MDI sind zahlreiche Methoden bekannt. Die Natur der störenden Farbkörper ist bisher jedoch nur unzureichend geklärt.

Die bislang bekannten Verfahren lassen sich in vier Gruppen unterteilen:
1. Verfahren, bei denen die als Ausgangsmaterial eingesetzten Di-und Polyamine der Diphenylmethanreihe (MDA) einer Behandlung und/oder Reinigung unterzogen wurden.
   Die EP-A 0 546 398 beschreibt ein Verfahren zur Herstellung von MDI, bei dem das als Edukt verwendete MDA vor der Phosgenierung angesäuert wird.
   Die EP-A 0 446 781 betrifft ein Verfahren zur Herstellung von MDI, bei dem das MDA zuerst mit Wasserstoff behandelt und anschließend einer Phosgenierung unterzogen wird, wobei ein helleres MDI erhalten wird.
   Die obengenannten Verfahren ergeben nur eine geringfügige Farbverbesserung, da die Farbkörper im MDI erfahrungsgemäß nicht nur aus bestimmten Nebenkomponenten der MDA-Herstellung bestehen, sondern auch aus Farbprecursoren resultieren, die durch Nebenreaktionen während der Phosgenierung gebildet werden.
2. Verfahrenstechnische Lösungen im Phosgenierungsprozeß
   Die US-A 5 364 958 betrifft ein Verfahren zur Herstellung von Isocyanaten, bei dem nach der Phosgenierung das Phosgen bei niedriger Temperatur vollständig entfernt wird und anschließend das Isocyanat in der Wärme mit HCl-Gas behandelt wird.
   In der DE-A-1981 7691 wird ein Verfahren zur Herstellung von MDI mit vermindertem Gehalt an chlorierten Nebenprodukten und verminderter Iodfarbzahl durch Einhaltung definierter Parameter in der Phosgenierungsreaktion beschrieben. Insbesondere ist hier die Einhaltung bestimmter Phosgen/HCl-Verhältnisse in der Reaktionsstufe erforderlich. Dieses Verfahren hat den Nachteil, dass eine Variation der Parameter in der Phosgenierung erschwert und dadurch die Qualität der Phosgenierung sehr anfällig wird. Durch die fehlende Flexibilität der Parameter in der Phosgenierung wird außerdem eine praktische Durchführung der Phosgenierung sehr schwierig und erfordert einen hohen technischen Aufwand.
   Eine andere Möglichkeit der Verbesserung der Farbe von Isocyanaten ist gemäß EP-B 1 187 808 die Verwendung von Phosgen mit niedrigen Gehalten an Brom- und/oder Jod.
   Verfahren der genannten Art versuchen zwar an der richtigen Stelle die Verfärbungen verursachenden Komponenten abzutrennen, sie sind jedoch sowohl aufgrund ihres hohen technischen Aufwands als auch in ihrem Farbaufhellungseffekt zu wenig effizient, da der Abbau von Farbvorläufern durch unvollständig ablaufende chemische Reaktionen nur in geringem Umfang erfolgt.
3. Zusatz von Farbaufhellungs-Additiven zum Isocyanat-Rohprodukt nach erfolgter Phosgenierung und vor der Aufarbeitung z.B. durch Destillation.
   Die EP-A 0 581 100 betrifft ein Verfahren zur Herstellung von Isocyanaten, bei dem nach der Phosgenierung ein chemisches Reduktionsmittel vor dem Abtrennen von Lösemittel zugegeben wird, wobei gemäß dieser Druckschrift ebenfalls helle Produkte erhalten werden.
   Gemäß der US-A 4 465 639 wird dem nach der Phosgenierung erhaltenen Rohprodukt zur Farbaufhellung Wasser zugesetzt. Zum gleichen Zweck beschreiben die EP-A 538 500, EP-A 0 445 602 und EP-A 0 467 125 den Zusatz von Carbonsäuren, Alkanolen bzw. Polyetherpolyolen nach der Phosgenierung.
   Die oben beschriebenen Verfahren zur Farbaufhellung sind zwar effizient, sie weisen jedoch Nachteile dahingehend auf, dass neben der Farbaufhellung die zugegebenen Additive Reaktionen mit den als Produkt anfallenden Isocyanaten eingehen und daraus beispielsweise in der Regel eine unerwünschte Verminderung des lsocyanatgehalts resultiert. Außerdem besteht die Gefahr der Bildung von unerwünschten Nebenprodukten im MDI.
4. Nachbehandlung des Endprodukts
   Die EP-A 0 133 538 beschreibt die Reinigung von Isocyanaten durch Extraktion, wodurch Fraktionen eines hellen MD1 erhalten werden.
   Die EP-A 0 561 225 betrifft ein Verfahren zur Herstellung von Isocyanaten oder Isocyanatgemischen, die laut dieser Schrift keine farbgebenden Komponenten aufweisen, wobei die Isocyanate nach der Phosgenierung der entsprechenden Amine einer Wasserstoffbehandlung bei einem Druck von 1 bis 150 bar und einer Temperatur von 100 bis 180°C unterworfen werden. Dabei werden gemäß den dort beschriebenen Beispielen Isocyanat-Endprodukte als solche oder in Form ihrer Lösungen in geeigneten Lösemitteln hydriert.
   Diese farbverbessernden Nachbehandlungen der Isocyanat-Endprodukte nach der vollständigen Abtrennung des Lösemittels bei erhöhter Temperatur sind ebenfalls wenig effizient, da durch die hohen Temperaturen, die bei der Aufarbeitung, insbesondere dem Abdestillieren des Lösemittels und ggf. von monomerem MDI (Diisocyanate) auftreten, bereits stabile Farbkörper entstanden sind, die chemisch nur noch schwer abzubauen sind.

Die Aufgabe der vorliegenden Erfindung besteht darin, ein einfaches und wirtschaftliches Verfahren zur Herstellung von hellen Isocyanaten, die keine oder nur geringe Mengen an farbgebenden Komponenten enthalten, zur Verfügung zu stellen. Dabei soll das Verfahren ohne die oben genannten Behandlungsschritte auskommen und dennoch zu hellen Isocyanaten, die zur Herstellung von Polyurethanen oder deren Vorläufern (z.B. Prepolymeren) geeignet sind, führen.

Die erfindungsgemäße Aufgabe konnte überraschenderweise dadurch gelöst werden, dass bei der Herstellung der Isocyanate Phosgen eingesetzt wird, das weniger als 100 ppm, bevorzugt weniger als 60 ppm, besonders bevorzugt weniger als 20 ppm an Schwefel in elementarer oder gebundener Form aufweist. Dabei bedeutet die Bereichsangabe "weniger als 100 ppm an Schwefel", dass im eingesetzten Phosgen weniger als 100 ppm an Schwefel bezogen auf das Gewicht des eingesetzten Phosgens enthalten sind.

Gegenstand der vorliegenden Erfindung ist ein Verfahren zur Herstellung von Isocyanaten durch Umsetzung eines Amins oder eines Gemischs aus zwei oder mehr Aminen mit Phosgen, dadurch gekennzeichnet, dass das eingesetzte Phosgen weniger als 100 ppm, bevorzugt weniger als 60 ppm, besonders bevorzugt weniger als 20 ppm an Schwefel in elementarer oder gebundener Form enthält.

Schwefel in elementarer Form ist Schwefel, der in Form von ausschließlich Schwefel enthaltenden Molekülen vorliegt. Unter Schwefel in gebundener Form werden Verbindungen oder Moleküle verstanden, die neben Schwefel noch weitere, von Schwefel verschiedene Atome enthalten wie beispielsweise Schwefelwasserstoff.

Das im Rahmen der vorliegenden Erfindung eingesetzte Phosgen enthält damit weniger als 100 ppm, bevorzugt weniger als 60 ppm, besonders bevorzugt weniger als 20 ppm an Schwefel in Form von elementarem Schwefel oder in Form von schwefelhaltigen Verbindungen.

Das erfindungsgemäße Verfahren führt dabei zu Isocyanaten, die ohne zusätzliche Verfahrensschritte zur Herstellung von Urethanverbindungen wie Polyurethanen oder deren Vorläufern (z.B. Prepolymeren) einsetzbar sind und die keine oder nur eine geringe Färbung aufweisen.

Das erfindungsgemäße Verfahren führt überraschenderweise zu hellen Isocyanaten, da bislang nicht bekannt war, dass bereits äußerst geringe Spuren von elementarem oder gebundenem Schwefel im Phosgen ausreichen, um die Produktfarbe der damit hergestellten Isocyanate in unerwünschter Weise zu beeinflussen.

Das zur Herstellung von Isocyanaten eingesetzte Phosgen weist in der Regel einen bestimmten Gehalt an elementarem oder gebundenem Schwefel auf. Der Gehalt an Schwefel im Phosgen resultiert dabei ganz wesentlich aus dem zur Phosgenherstellung eingesetzten Kohlenmonoxid (CO), das je nach Herkunft einen bestimmten Anteil an Schwefel aufweist. Der Gehalt des Schwefels resultiert wieder überwiegend aus dem Schwefel-Gehalt der zur CO-Herstellung verwendeten Rohstoffe.

Das im erfindungsgemäßen Verfahren eingesetzte Phosgen mit niedrigem Gehalt an Schwefel kann auf verschiedene, dem Fachmann bekannte Arten hergestellt werden. Eine Möglichkeit einen niedrigen Gehalt an Schwefel im Phosgen zu garantieren, ist beispielsweise die Verwendung von Ausgangsverbindungen bei der Phosgenherstellung, die bereits einen entsprechend niedrigen Gehalt an Schwefel aufweisen. Insbesondere bietet sich hierbei die Verwendung von CO mit einem entsprechend niedrigen Gehalt an Schwefel an. Verfahren zur Herstellung von CO mit einem niedrigen Gehalt an Schwefel sind in der Fachwelt bekannt.

So kann beispielsweise CO eingesetzt werden, das durch Kohlevergasung, Dampf-Reforming, CO₂-Reforming, partielle Oxidation von Kohlenwasserstoffen oder andere Verfahren gewonnen wurde. CO kann ebenfalls durch Abtrennung aus CO-haltigen Gasgemischen gewonnen werden.

Derartige Verfahren zur Herstellung bzw. Gewinnung von CO werden z. B. in Chemische Technik (Hrsg.: Dittmeyer, Keim, Kreysa, Oberholz), 5. Aufl., Bd. 4, Seite 981-1007 beschrieben.

Grundsätzlich kann im Rahmen der vorliegenden Erfindung jedes CO eingesetzt werden, das die o.g. Spezifikation erfüllt, d.h., weniger als 100 ppm, bevorzugt weniger als 60 ppm, besonders bevorzugt weniger als 20 ppm an Schwefel aufweist.

Der erforderliche niedrige Schwefelgehalt im CO wird erreicht, indem für die CO-Herstellung bzw. -Gewinnung Rohstoffe eingesetzt werden, die praktisch schwefelfrei sind oder einen ausreichend niedrigen Schwefelgehalt aufweisen. Dabei ist es unerheblich, wie die Schwefelfreiheit bzw. der ausreichend niedrige Schwefelgehalt der für die CO-Herstellung bzw. -Gewinnung eingesetzten Rohstoffe erreicht wird.

Eine andere Möglichkeit, den erforderlichen niedrigen Schwefelgehalt im CO zu erreichen, besteht in der Entfernung von in elementarer oder gebundener Form vorliegendem Schwefel aus dem einzusetzenden CO. In der Literatur sind zahlreiche Verfahren beschrieben die diesem Zweck dienen. Beispielhaft sei z.B. erwähnt die Entfernung von schwefelhaltigen Verunreinigungen wie z.B. H₂S gemäß US-A1-2005/0025693 dadurch, dass der CO-haltige Gasstrom mit Aktivkohle, die mit einem Metalloxid impregniert wurde, in Kontakt gebracht wird. Eine andere Möglichkeit ist z.B. gemäß DE-A1-103 01 434 die Konvertierung anorganischer und organischer Schwefelverbindungen in Gegenwart von Wasserdampf an einem Aluminiumoxid-Kontakt bei erhöhter Temperatur und Überleiten des erhaltenen Gasgemisches über eine Eisenhydroxid-haltige Masse in Gegenwart von Wasserdampf und definierter Mengen an Sauerstoff. DE-A1-103 01 434 zitiert noch weitere Möglichkeiten, CO von schwefelhaltigen Verunreinigungen zu befreien.

Das so erhältliche, weitgehend schwefelfreie CO kann anschließend im Rahmen üblicher und bekannter Prozesse wie sie z.B. in Ullmanns Enzyklopädie der industriellen Chemie, 3. Aufl., Bd. 13, Seite 494-500 beschrieben sind, zu Phosgen umgesetzt werden.

Ein weiterer Weg, Phosgen mit einem niedrigen Gehalt an elementarem oder gebundenem Schwefel zu erhalten, ist die Abtrennung von elementarem oder gebundenem Schwefel aus dem Phosgen selbst. Auch hier können im Prinzip wieder alle gängigen Trennverfahren eingesetzt werden, beispielsweise Destillation, Adsorption und dergleichen. Für das erfindungsgemäße Verfahren ist letztlich ausschließlich die Einhaltung der oben genannten Obergrenze für die Konzentration an elementarem oder gebundenem Schwefel entscheidend.

Die nach dem erfindungsgemäßen Verfahren stattfindende Isocyanatherstellung wird durch Umsetzung eines Amins oder eines Gemischs aus zwei oder mehr Aminen mit Phosgen, bevorzugt im Überschuss von Phosgen, durchgeführt. Anwendbar sind grundsätzlich alle dem Fachmann bekannten Verfahren, bei denen ein primäres Amin oder ein Gemisch aus zwei oder mehr primären Aminen mit Phosgen unter Bildung einer oder mehrerer Isocyanatgruppen umgesetzt wird.

In einer bevorzugten Ausführungsform des erfindungsgemäßen Verfahrens wird die Umsetzung des Amins oder des Gemischs aus zwei oder mehr Aminen mit dem Phosgen in einem Lösemittel oder einem Gemisch aus zwei oder mehr Lösemitteln durchgeführt.

Als Lösemittel können alle für die Herstellung von Isocyanaten geeigneten Lösemittel eingesetzt werden. Vorzugsweise sind dies inerte aromatische, aliphatische oder alicyclische Kohlenwasserstoffe bzw. deren halogenierte Derivate. Beispiele für solche Lösemittel sind aromatische Verbindungen wie Mono- oder Dichlorbenzol, beispielsweise o-Dichlorbenzol, Toluol, Xylole, Naphthalinderivate wie Tetralin oder Decalin, Alkane mit etwa 5 bis etwa 12 C-Atomen wie Hexan, Heptan, Octan, Nonan oder Decan, Cycloalkane wie Cyclohexan, inerte Ester und inerte Ether wie Ethyl- oder Butylacetat, Tetrahydrofuran, Dioxan oder Diphenylether.

Als Amine eignen sich prinzipiell alle primären Amine die in geeigneter Weise mit Phosgen zu Isocyanaten reagieren können. Geeignet sind prinzipiell alle linearen oder verzweigten, gesättigten oder ungesättigten aliphatischen oder cycloaliphatischen oder aromatischen primären Mono-, Di- oder Polyamine, sofern diese mit Phosgen zu Isocyanaten umgesetzt werden können. Beispiele für geeignete Amine sind 1,3-Propylendiamin, 1,4-Butylendiamin, 1,5-Pentamethylendiamin, 1,6-Hexamethylendiamin und die entsprechenden höheren Homologen dieser Reihe, Isophorondiamin (IPDA), Diaminodicyclohexylmethan, Cyclohexyldiamin, Cyclohexylamin, Anilin, Phenylendiamin, p-Toluidin, Naphthalindiamin und insbesondere 1,5-Naphtylendiamin, Toluylendiamin und insbesondere 2,4- oder 2,6-Toluylendiamin oder deren Gemische, Di- und Polyamine der Diphenylmethanreihe und insbesondere 4,4'-, 2,4'- oder 2,2'-Diphenylmethandiamin oder deren Gemische sowie höhermolekulare isomere, oligomere oder polymere Derivate der obengenannten Amine und Polyamine. Im Rahmen einer bevorzugten Ausführungsform der vorliegenden Erfindung wird als Amin ein Amin der Diphenylmethan-Reihe oder ein Gemisch aus Di- und/oder Polyaminen der Diphenylmethanreihe eingesetzt.

Nach Durchlaufen des erfindungsgemäßen Verfahrens liegen die obengenannten Verbindungen in Form der entsprechenden Isocyanate vor, z.B. als 1,6-Hexamethylendiisocyanat, Isophorondiisocyanat, Diisocyanatodicyclohexylmethan, Cyclohexylisocyanat, Cyclohexyldiisocyanat, Phenylisocyanat, Phenylendiisocyanat, 4-Toluylisocyanat, Naphthalindiisocyanat und insbesondere 1,5-Naphthylendiisocyanat, Toluylendiisocyanat und insbesondere 2,4- oder 2,6-Toluylendiisocyanat oder deren entsprechende Gemische, Di- und Polyisocyanate der Diphenylmethanreihe und insbesondere 4,4'-, 2,4'- oder 2,2'-Diphenylmethandiisocyanat oder Gemische aus zwei oder mehr davon, sowie als höhermolekulare oligomere oder polymere Derivate der obengenannten Isocyanate oder als Gemische aus zwei oder mehr der genannten Isocyanate oder Isocyanatgemische.

In einer bevorzugten Ausführungsform der vorliegenden Erfindung werden als Amine die isomeren, primären Diphenylmethandiamine (MDA) bzw. deren oligomere oder polymere Derivate eingesetzt, d.h., die Di- und Polyamine der Diphenylmethan-Reihe. Diphenylmethandiamin, dessen Oligomere oder Polymere werden beispielsweise durch Kondensation von Anilin mit Formaldehyd erhalten. Auch solche Oligo- oder Polyamine oder deren Gemische werden im Rahmen einer bevorzugten Ausführungsform der Erfindung eingesetzt.

Die Umsetzung des schwefelarmen oder sogar schwefelfreien Phosgens mit einem der obengenannten Amine oder einem Gemisch aus zwei oder mehr solcher Amine in dem erfindungsgemäßen Verfahren, kann kontinuierlich oder diskontinuierlich in einer oder in mehreren Stufen erfolgen. Wird eine einstufige Umsetzung durchgeführt, so erfolgt diese Umsetzung vorzugsweise bei 60 bis 200°C, beispielsweise bei 130 bis 180°C.

In einer weiteren Ausführungsform der Erfindung kann die Umsetzung zweistufig durchgeführt werden. Hierbei wird in einer ersten Stufe die Umsetzung des Phosgens mit dem Amin oder dem Gemisch aus zwei oder mehr Aminen bevorzugt bei einer Temperatur von zwischen 0 und 130°C, besonders bevorzugt 20 bis 110°C, ganz besonders bevorzugt 40 bis 70°C durchgeführt, wobei für die Reaktion zwischen Amin und Phosgen bevorzugt eine Zeitspanne von 1 min bis 2 h eingeräumt wird. Anschließend wird in einer zweiten Stufe bevorzugt während einer Zeitspanne von 1 min bis 5 h, besonders bevorzugt von 1 min bis 3 h, die Temperatur bevorzugt auf 60 bis 190°C, besonders bevorzugt 70 bis 170°C, erhöht.

In einer bevorzugten Ausführungsform der Erfindung wird die Umsetzung in zwei Stufen durchgeführt.

Während der Umsetzung kann bevorzugt ein erhöhter absoluter Druck angelegt werden, der bevorzugt 100 bar oder weniger, besonders bevorzugt 1 bar bis 50 bar, ganz besonders bevorzugt 2 bar bis 25 bar, insbesondere ganz besonders bevorzugt 3 bar bis 12 bar, beträgt. Die Umsetzung kann jedoch auch drucklos erfolgen.

In einer weiteren bevorzugten Ausführungsform der Erfindung wird daher bei Umgebungsdruck, d.h. bei in der Regel etwa 1 bar absolut gearbeitet. In einer weiteren bevorzugten Ausführungsform kann zudem bei gegenüber dem Umgebungsdruck reduziertem Druck gearbeitet werden.

Überschüssiges Phosgen wird vorzugsweise im Anschluss an die Umsetzung bei einer bevorzugten Temperatur von 50 bis 200°C entfernt. Die Entfernung der restlichen Spuren des Lösemittels erfolgt vorzugsweise unter vermindertem Druck. Bevorzugt beträgt der absolute Druck 500 mbar oder weniger, besonders bevorzugt weniger als 100 mbar. Im Allgemeinen werden dabei die verschiedenen Komponenten in der Reihe der Siedepunkte abgetrennt, wobei auch die Abtrennung von Gemischen der verschiedenen Komponenten in einer einzigen Verfahrensstufe möglich ist.

Die Erfindung wird nachfolgend durch Beispiele näher erläutert.

### Beispiele:

### Allgemeine Arbeitsvorschrift:

### Ausgangsmaterialien:

- MDA, erhalten durch sauer katalysierte Kondensation von Anilin mit Formaldehyd, bestehend im wesentlichen aus 4,4'-MDA, 2,4'-MDA, 2,2'-MDA (2-Kern-MDA) sowie höheren Homologe und Isomeren, Gehalt an 2-Kern-MDA 61,0 Gew.-%
- Chlorbenzol wasserfrei
- Phosgen

50 g des MDA werden in 255 ml Chlorbenzol gelöst, auf 55°C erwärmt und innerhalb von 10 s unter intensivem Rühren zu einer auf 0°C temperierten Lösung von 105 g Phosgen in 310 ml Chlorbenzol gegeben. Der Schwefelgehalt des eingesetzten Phosgens kann der nachfolgenden Tabelle entnommen werden. Die Suspension wird unter Durchleiten von Phosgen innerhalb von 45 min auf 100°C und anschließend während 10 Min auf Rückflusstemperatur aufgeheizt. Nach weiteren 10 min bei dieser Temperatur wird das Lösungsmittel unter vermindertem Druck bis zu einer Sumpftemperatur von 100°C abdestilliert. Das rohe Isocyanat wird anschließend in einer Destillationsapparatur bei einem Druck von 4 bis 6 mbar mittels eines Heißluftgebläses bis zum ersten Produktübergang erhitzt und daraufhin innerhalb von 5 bis 10 min auf Raumtemperatur abgekühlt. Von dem so erhaltenen Isocyanat wird 1,0 g in Chlorbenzol gelöst und mit Chlorbenzol zu 50 ml verdünnt. Die Extinktion der so erhaltenen Lösung wird bei den beiden Wellenlängen 430 nm und 520 nm bestimmt. Als Messgerät wird ein Photometer Dr. Lange LICO 300 eingesetzt. Die Ergebnisse sind in der folgenden Tabelle zusammengefasst.

Aus dem Vergleich der Beispiele 1 bis 4 mit den Vergleichsbeispielen 1 und 2 ist ersichtlich, dass die Verwendung von schwefelarmem Phosgen zu Isocyanaten mit niedrigen Farbwerten führt.

| | **ppm S** | **E 430** | **E 520** |
|---|---|---|---|
| **Beispiel 1** | 0 | 0,100 | 0,008 |
| **Beispiel 2** | 20 | 0,105 | 0,016 |
| **Beispiel 3** | 60 | 0,116 | 0,024 |
| **Beispiel 4** | 80 | 0,130 | 0,030 |
| **Vergleichsbeispiel 1** | 250 | 0,162 | 0,060 |
| **Vergleichsbeispiel 2** | 500 | 0,241 | 0,121 |

## Patentansprüche

1. Verfahren zur Herstellung von Isocyanaten durch Umsetzung eines Amins oder eines Gemischs aus zwei oder mehr Aminen mit Phosgen, **dadurch gekennzeichnet, dass** das eingesetzte Phosgen weniger als 100 ppm Schwefel in elementarer oder gebundener Form enthält.

2. Verfahren nach Anspruch 1, **dadurch gekennzeichnet, dass** das eingesetzte Phosgen weniger als 60 ppm Schwefel in elementarer oder gebundener Form enthält.

3. Verfahren nach Anspruch 1, **dadurch gekennzeichnet, dass** das eingesetzte Phosgen weniger als 20 ppm Schwefel in elementarer oder gebundener Form enthält.

4. Verfahren nach einem der Ansprüche 1 bis 3, **dadurch gekennzeichnet, dass** die Umsetzung in einem Lösemittel durchgeführt wird.

5. Verfahren nach einem der Ansprüche 1 bis 4, **dadurch gekennzeichnet, dass** Di- und Polyamine der Diphenylmethan-Reihe eingesetzt werden.

6. Verfahren nach einem der Ansprüche 1 bis 4, **dadurch gekennzeichnet, dass** Toluylendiamin eingesetzt wird.

7. Verfahren nach einem der Ansprüche 1 bis 4, **dadurch gekennzeichnet, dass** Hexamethylendiamin oder Isophorondiamin oder Diaminodicyclohexylmethan eingesetzt wird.

## Claims

1. Process for preparing isocyanates by reacting an amine or a mixture of two or more amines with phosgene, **characterized in that** the phosgene used contains less than 100 ppm of sulphur in elemental or bound form.

2. Process according to Claim 1, **characterized in that** the phosgene used contains less than 60 ppm of sulphur in elemental or bound form.

3. Process according to Claim 1, **characterized in that** the phosgene used contains less than 20 ppm of sulphur in elemental or bound form.

4. Process according to any of Claims 1 to 3, **characterized in that** the reaction is performed in a solvent.

5. Process according to any of Claims 1 to 4, **characterized in that** di- and polyamines of the diphenylmethane series are used.

6. Process according to any of Claims 1 to 4, **characterized in that** tolylenediamine is used.

7. Process according to any of Claims 1 to 4, **characterized in that** hexamethylenediamine or isophoronediamine or diaminodicyclohexylmethane is used.

## Revendications

1. Procédé de fabrication d'isocyanates par réaction d'une amine ou d'un mélange de deux amines ou plus avec du phosgène, **caractérisé en ce que** le phosgène utilisé contient moins de 100 ppm de soufre sous forme élémentaire ou reliée.

2. Procédé selon la revendication 1, **caractérisé en ce que** le phosgène utilisé contient moins de 60 ppm de soufre sous forme élémentaire ou reliée.

3. Procédé selon la revendication 1, **caractérisé en ce que** le phosgène utilisé contient moins de 20 ppm de soufre sous forme élémentaire ou reliée.

4. Procédé selon l'une quelconque des revendications 1 à 3, **caractérisé en ce que** la réaction est réalisée dans un solvant.

5. Procédé selon l'une quelconque des revendications 1 à 4, **caractérisé en ce que** des di- et polyamines de la série du diphénylméthane sont utilisées.

6. Procédé selon l'une quelconque des revendications 1 à 4, **caractérisé en ce que** de la toluylènediamine est utilisée.

7. Procédé selon l'une quelconque des revendications 1 à 4, **caractérisé en ce que** de l'hexaméthylènediamine ou de l'isophoronediamine ou du diaminodicyclohexylméthane est utilisé.
